**Europäisches Patentamt**

**European Patent Office**

**Office européen des brevets**

(19)

(11) Publication number: **0 112 166**
**B1**

(12) # EUROPEAN PATENT SPECIFICATION

(45) Date of publication of patent specification: **24.08.88**

(51) Int. Cl.⁴: **G 01 N 33/52, C 12 Q 1/00**

(21) Application number: **83307601.1**

(22) Date of filing: **14.12.83**

(54) **Method for quantitative analysis using analytical element sheet.**

(30) Priority: **14.12.82 JP 218910/82**

(43) Date of publication of application:
**27.06.84 Bulletin 84/26**

(45) Publication of the grant of the patent:
**24.08.88 Bulletin 88/34**

(84) Designated Contracting States:
**DE GB**

(56) References cited:
**GB-A-2 069 131**
**GB-A-2 069 132**
**US-A-4 248 829**

(73) Proprietor: **FUJI PHOTO FILM CO., LTD.**
**210 Nakanuma Minamiashigara-shi**
**Kanagawa-ken, 250-01 (JP)**

(72) Inventor: **Kitajima, Masao**
**C/O Fuji Photo Film Co. Ltd. 3-11-46 Senzui**
**Asaka-shi Saitama (JP)**
Inventor: **Iwata, Yuzo**
**C/o Fuji Photo Film Co. Ltd. 3-11-46 Senzui**
**Asaka-shi Saitama (JP)**
Inventor: **Amano, Yoshikazu**
**C/o Fuji Photo Film Co. Ltd. 3-11-46 Senzui**
**Asaka-shi Saitama (JP)**
Inventor: **Kondo, Asaji**
**C/o Fuji Photo Film Co. Ltd. 3-11-46 Senzui**
**Asaka-shi Saitama (JP)**

(74) Representative: **Arthur, Bryan Edward et al**
**Withers & Rogers 4 Dyer's Buildings Holborn**
**London EC1N 2JT (GB)**

Courier Press, Leamington Spa, England.

# 0 112 166

**Description**

Background of the invention
Field of the invention

This invention relates to a method for quantitative analysis of a substance to be analyzed (analyte) in a liquid sample using an analytical element sheet. More particularly, the present invention relates to a method for quantitative analysis of an analyte in a blood sample which comprises applying a certain amount of a blood sample onto an analytical element in the form of a sheet comprising a reagent-retaining layer provided on a transparent support and photometrically measuring a change of color density or a rate of the change of color density from the transparent support side to determine quantitatively the amount of the analyte in the blood sample.

Description of prior arts

An analytical element in the form of a sheet comprising at least one reagent-containing layer provided on a transparent support is disclosed in, for instance, Japanese Patent Publication No. 49(1974)-53888; Japanese Patent Provision Publications No. 50(1975)-137192, No. 51(1976)-40191, No. 52(1977)-3488, No. 53(1978)-131089, No. 54(1979)-101398, No. 57(1982)-66359, No. 55(1980)-164356, No. 57(1982)-148250, and No. 56(1981)-24576; Japanese Utility Model Provisional Publication No. 57(1982)-42951; US—A—3,298,789 and 3,395,082; Clinical Chemistry, 24, 1335—1350 (1978) ibid., 27, 1287—1290 (1981); GB—A—2069131A and 2069132A; and US—A—4248829.

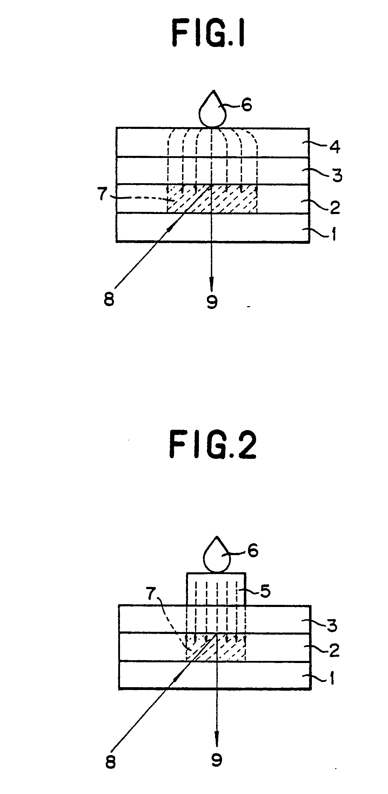

Representative structures of the analytical element sheet disclosed in the above-mentioned publications are illustrated in Fig. 1 and Fig. 2 in the form of section views, in which a transparent support 1 is provided with at least one reagent-retaining layer (or reagent layer) 2 thereon, and a spreading layer 4 or a patch having a definite surface area 5 is arranged on the reagent layer 2. If necessary, a light-shielding layer or a light-reflecting layer 3, as well as a variety of other functional layers such as a barrier layer and a detection layer, can be incorporated into the element.

The method for quantitative analysis of an analyte in a liquid sample can be carried out in the following manner, using an analytical element having the structure of Fig. 1.

The liquid sample is applied (spotted or placed) onto the spreading layer 4. The applied sample spreads rapidly to form a circle on the spreading layer 4 and subsequently permeates into the reagent layer 2 to react with a reagent selectively reactive to the analyte which is previously incorporated into the reagent layer, forming a dye. The amount of thus formed dye is proportional to the content of the analyte. Accordingly, the quantitative analysis of the analyte is accomplished by photometrically measuring the amount of the formed color or the rate of the color formation from the support side.

The above-described method is based on the fact that the applied sample spreads on the spreading layer to form a circle whose area is substantially proportional to the amount of the applied sample, namely, metering effect. Alternatively, the analytical element illustrated in Fig. 2 utilizes a patch having a definite surface area whereby the color-forming reaction is defined to take place within the area corresponding to the bottom of the patch.

These analytical elements in the form of sheet are available on the market or tentatively employed, and are of value particularly for quantitative analyses of chemical species in body fluids such as blood and urine.

It is well known that a blood sample such as plasma, serum or whole blood varies per each sample in certain properties such as the contents of concrete materials, protein concentration (content), and viscosity. Also known is that a blood sample varies in fluidity and other properties depending upon the kind of additives and the period of storage.

The aforementioned analytical element sheet, however, cannot satisfactorily cope with the variation on the properties. For instance, the analytical element gives substantially quantitative results in the analysis of regular bloods, while gives unsatisfactory results in the analysis of bloods whose properties are deviated from the regular ranges. Accordingly, it has been noted that satisfactory results are hardly obtainable in the analysis of blood samples whose properties such as the contents of concrete materials and viscosities, are deviated from the regular ranges, as far as the analysis is performed in the conventional manner which is based on determination of the analyte content from a single value such as a value of color concentration or a rate of the color change observed on the sample-applied analytical element through the transparent support.

As a result of a variety of experimental studies, the present inventors have discovered that satisfactory analytical results can be given even in the analysis of blood samples whose properties are deviated from the regular ranges by, in the first place, choosing as a fundamental value a value obtained in a conventional manner in which the change of color density or the rate of the change of color density produced on the analytical element by the application of a liquid sample such as whole blood or serum is photometrically measured from the transparent support side; in the second place, choosing as a supplementary value, at least one value selected from the group consisting of a value obtained by photometrically measuring a color density or a shape (size, diameter, etc.) of the colored portion produced on the surface of the analytical element (the surface of the spreading layer or the patch) onto which the liquid sample has been applied, and a value obtained by photometrically measuring a shape of the colored portion from the

2

transparent support side; and then correcting the above-mentioned fundamental value by applying thereto a certain correction value obtained using the supplementary value as at least one parameter. It is not clear to which influential factor of the properties of the sample the supplementary value corresponds. It is thought, however, that the supplementary value corresponds to hematocrit value and fluidity for whole blood sample, while it corresponds to protein content or fluidity for plasma or serum sample. References to blood herein include whole blood, plasma and serum.

Summary of the invention

According to the present invention there is provided a method (1) for quantitative analysis of an analyte contained in a blood sample which comprises applying a certain amount of blood sample onto an analytical element in the form of a sheet comprising a reagent-retaining layer provided on a transparent support and photometrically measuring a change of color density or a rate of the change of color density from the transparent support side to determine quantitatively the amount of the analyte in the blood sample, characterised in that the method comprises the steps of:

choosing, as a fundamental value, a said value determined by measuring photometrically from the transparent support side the change of color density or the rate of the change of color density produced on the analytical element by the application of the blood sample;

choosing and determining, as a supplementary value, at least one value selected from a value obtained by photometrically measuring a color density or a shape of the colored portion on the surface of the same element onto which the blood sample has been applied, and a value obtained by photometrically measuring a shape of the colored portion from the transparent support side; and

correcting said fundamental value by applying thereto a correction value determined from a predetermined relationship between the supplementary value and a factor of the blood sample influencing the supplementary value and the fundamental value.

With the present invention errors in measurement inherently observed in the analysis employing a dry analytical element sheet, which stem from various influential factors of a liquid sample, particularly certain factors influencing the spreading behaviour of the liquid sample, can be accurately and readily corrected.

The method may be employed:

for accurately and readily correcting the error in measurement arising from hematocrit that is an influential factor in the analysis of whole blood sample and further for accurately and readily correcting the error in measurement arising from the presence of proteins that is an influential factor in the analysis of a liquid plasma or serum sample;

for appropriately correcting errors in measurement stemmed from other influential factors such as variation and difference of the applied volume or the sample applied to the element;

for quantitative analysis using a dry analytical element sheet which is substantially free from problems inherently occurring in the use of said element sheet, for instance, a measurable sample is under certain restrictions, whereby providing an analytical method employable for analyses of a wide variety of samples.

Preferred features of the invention are as follows:

(2) The above-defined method (1), in which determining the fundamental value is performed prior to determining the supplementary value.

(3) The above-defined method (1), in which determining the fundamental value and determining the supplementary value are performed substantially at the same time.

(4) The above-defined method (1), in which determining the supplementary value is performed prior to determining the fundamental value.

(5) The above-defined method (1), in which the analytical element comprises a spreading layer provided on the reagent-retaining layer and the blood sample is applied onto said spreading layer.

(6) The above-defined method (5), in which the analytical element comprises a light-shielding layer provided between the reagent-retaining layer and the spreading layer.

(7) The above-defined method (1), in which the analytical element comprises a patch having a definite surface area and the blood sample is applied onto said patch.

(8) The above-defined method (7), in which the analytical element comprises a light-shielding layer provided between the reagent-retaining layer and the patch.

Brief description of drawings

Fig. 1 is a schematical section view of a typical known analytical element sheet (film) having a porous spreading layer, and Fig. 2 is a schematical section view of a typical known analytical element sheet carrying a porous patch having a definite surface area. In these figures is schematically illustrated a method involving the application of a liquid sample onto the analytical element and the reflection photometry through the transparent support on the colored portion of the reagent layer of the element to obtain a reflection optical density (or reflection density, which corresponds to the fundamental value in the method of the present invention).

In Fig. 1 and Fig. 2, respective numbers indicate the following:

1: transparent support, 2: reagent-retaining layer (or reagent layer), 3: light-reflecting layer, 4: porous spreading layer, 5: porous patch having a definite (or fixed) surface area, 6: a drop of liquid sample, 7: colored portion of reagent-retaining layer, 8: light source for reflection photometry (not shown), and 9:

3

photometric measuring apparatus (not shown), in which the area 7 for the colored portion is schematically indicated using hatching of broken lines given within the reagent-retaining layer 2, and the flow of the applied liquid sample is also schematically indicated by the arrow of broken line.

Fig. 3 is a graph indicating a correlation between the logarithmic centrifugal hematocrit value of whole blood and the reflection density of the whole blood remaining on the spreading layer given in Example 1.

Fig. 4 is a graph indicating a correlation between the surface reflection density of residual whole blood given in Example 1 and the deviation defined in Example 1, in which the lines A and B indicate the correlations on a NaF-containing whole blood sample and a no-NaF-containing whole blood sample, respectively.

Fig. 5 is a graph indicating a correlation between the hematocrit value and the spread area of the whole blood sample given in Example 2, in which the circle and triangle mean values given upon measurement of the whole blood samples having glucose contents of 118 mg/dl and 254 mg/dl, respectively.

Fig. 6 is a graph indicating a correlation between the hematocrit value and the area of colored portion produced on the reagent layer given in Example 3, in which the circle and triangle have the same meanings as defined in the description for Fig. 5.

Fig. 7 is a graph indicating a correlation between the hematocrit value and the diameter of a circular spreading portion of the whole blood sample given in Example 4.

Fig. 8 is a graph indicating a correlation between the hematocrit value and the reflection density of a filmy layer consisting essentially of red blood corpuscle and residing on the surface of the patch having a definite surface area given in Example 7.

Fig. 9 is a graph indicating a correlation between the hematocrit value and the reflection density of the colored portion measured through the transparent support (lower-side curve) and a correlation between the hematocrit value and area of the colored portion measured through the transparent support (upper-side curve).

Detailed description of the invention

In the method of the present invention, the measurement for determining the fundamental value is performed photometrically using visible rays, ultraviolet rays or infrared rays. The measurement can be done on determination of the optical density of reflecting light, fluorescence or emission. These measurements can be performed in known manners disclosed in the aforementioned patent publications and other texts. The procedures or operations for determining the fundamental value can be in principle done by the procedures or operations known in the art of quantitative analysis of an analyte contained in a liquid sample using a dry analytical element sheet. The fundamental value can be: a value of change determined by the known end point method; a rate of the change or a rate of an initial change determined by the known reaction rate method; or one of other values determined by other methods.

The supplementary value can be determined by photometric measurement utilizing visible rays, ultraviolet rays, infrared rays or fluorescence. In which the measurement can be likewise performed by a known method such as the one-point measuring method or a scanning method for detecting the density of formed color or the shape of the colored portion. The supplementary value can be also determined in other known methods such as scanning methods utilizing electron beam or ultrasonic wave.

The method for determining the supplementary value is further described below. Furthermore, the method for correcting the fundamental value using a correction value given from a certain relationship or correlation between the supplementary value and a certain influential factor is also described in detail.

Representative examples of the procedure for measuring the reflection density of a liquid sample-applied surface employable in the method of the present invention are given hereunder.

The liquid sample is applied (spotted or placed) in the form of a drop onto the analytical element sheet and immediately spreads therein. Although the spreading rate (speed) and the spread area both vary depending on various factors such as viscosity and amount of concrete species (e.g., hematocrit value) of the liquid sample, and amounts of lipids and proteins contained therein, these values are considered to vary depending predominantly upon the hematocrit value in such specific sample as whole blood. Other representative influential factors include structure and conditions of a surface of the analytical element to which the liquid sample is applied (the spreading layer, the patch having a definite surface area, or the reagent-retaining layer). Accordingly, the following description can be given on an analytical element in the form of sheet having a spreading layer.

The liquid sample applied onto the spreading layer spreads therein generally in the form of a circle or an oval (termed "spread circle") due to influence of a certain combination of the above-mentioned factors.

The measurement on reflection density of the spread circle is generally carried out by means of a reflection optical densitometer. However, careful attention should be given to certain procedures in the measurement for attaining accurate reflection measurement. For example, the diameter of a beam applied to the spread circle has to be smaller than the minimum diameter of a spread circle to be measured, because a correct parameter is given only by measuring a predetermined specific circle irrespective of variation of the spread area of the sample. More specifically, the measurement is preferably done on the central area (generally, having a diameter in the range of 1—5 mm) of the spread circle.

In the present invention, the representative procedures for the measurement on the spread area of the liquid sample applied onto an analytical element is described below in detail. ·

**0 112 166**

As described above, the spread area generally is in the form of a circle or an oval similar to the circle. In the case that the analysis is performed on a strongly colored sample such as whole blood, a contour of the colored area is made so clear as to provide a great photometric difference between the spread area and the non-spread area on the analytical element. In such a case, the spread area is determined by measuring diameters of the spread circle by the known scanning procedure which comprises measuring reflectance of the surface of the element moving horizontally at a constant rate under radiation of a narrow beam from the upper side.

The beam applicable for the above-mentioned purpose can be an electromagnetic wave employable in the conventional scanning procedure, for instance, white light, light of a restricted wavelength region, electron beam, ultrasonic wave, and infrared rays. These beams can be chosen and employed depending on the purpose of measurement. The movement of the analytical element can be naturally replaced with movement of the beam. In the case of using a light as the beam, a beam sensor having a small-sized amplifier or an optical fiberscope-type beam sensor equipped with an optical fiber is preferably employed for reducing the size of the apparatus.

Otherwise, the measurement of the spread area can be done by means of a beam sensor equipped radially with a great number of heads with narrow straight beams. In this embodiment, no scanning procedure is required, and the beam head, sensor and analytical element are fixed in the predetermined position.

The above-described scanning method, as well as the method using plural beam heads, can be utilized for measuring the diameter of the spread circle. Alternatively, the whole spread area can be determined by measuring reflectance of the spreading material to be measured (color of red blood corpuscle for whole blood or optical density of color for formed color) through uniform circular exposure corresponding to the photometric characteristics of the spreading material. However, since the optical density of formed color varies with the sample in the last-mentioned method, a certain threshold value should be set for the desired detection.

The supplementary value can be determined by the end point method for measuring an amount of change, the reaction rate method for measuring a rate of the change or a rate of an initial change, or other known methods. Generally, the determination is satisfactorily carried out by measurement of the amount of change.

The determination of the supplementary value can be done before or after the determination of the fundamental value. Otherwise, the determinations of the supplementary value and fundamental value can be performed substantially at the same time, which means that the periods for both determination procedures can overlap each other.

The supplementary value can be generally determined by measuring a single value indicating a parameter for one of the aforementioned influential factors. However, if required, two or more values can be measured for supplementing one or more parameters originating from the same or different influential factors.

Representative examples of the influential factors include viscosity, color, and amount of applied liquid sample (variation or error in the amount). In the case that the liquid sample is whole blood, the influential factors may consist essentially of hematocrit, fluidity, viscosity and protein and lipid contained therein. In the case that the liquid sample is plasma or serum, the influential factors may consist essentially of fluidity, viscosity, color, protein and lipid and preservative contained therein.

In carrying out the method of the present invention, a correlation (or relationship) between the supplementary value and the influential factor is beforehand determined to set a correction value in relation to the supplementary value.

The procedures for setting the correction value in relation to the supplementary value based on the correlation between the supplementary value determined in the above-described manners and the influential factor and then correcting the fundamental value by the use of the correction value, are described below.

One embodiment includes firstly determining a relationship or correlation between a spread area of a liquid sample on the spreading layer or a reflection density of a filmy residue of a liquid sample remaining on the spreading layer, the patch having a definite surface area, and the amount of a colored substance contained in the liquid sample, obtaining a correction value from a supplementary value utilizing the so determined correlation chart or a correlation table, and finally correcting the fundamental value by applying thereto the correction value. An alternative embodiment includes inputting a measured value (output) of the spread area or a reflection density of the residue of an applied liquid sample to an operation device or an operation circuit storing or memorizing as the fundamental value a reflection value separately measured on a colored area through the transparent support, that is, from the transparent support side of the analytical element, whereby obtaining through calculation a correction value corresponding to the supplementary value in the oepration device or operation circuit to directly correct the fundamental value. The calculating process can be carried out by a procedure comprising addition or subtraction of the correction value to or from the fundamental value, a procedure comprising multiplication or division of the fundamental value by the correction value, or any other known process.

The present invention shall be further described by the following examples.

5

# 0 112 166

Example 1

A solution of a reagent layer having the composition set forth below for quantitative analysis of glucose contained in blood was coated over a transparent polyethylene terephthalate (PET) film (support, thickness: 185 μm) having a gelatin subbing layer, and the coated layer was dried to give a dry reagent layer of 15 μm thick.

Composition

| | |
|---|---|
| Peroxidase | 25,000 IU |
| 1,7-Dihydroxynaphthalene | 5 g |
| 4-Aminoantipyrine | 5 g |
| Gelatin | 200 g |
| Nonion HS 210 (trademark of Nippon Oil & Fats Co., Ltd., Japan, polyoxyethylene nonylphenyl ether surfactant) | 2 g |

On the reagent layer was provided a light-shielding layer of approx. 15 μm thick (dry basis) consisting of 8 g of powdery titanium dioxide carrying 0.2 g of Nonion HS 210 and 50,000 IU of glucose oxidase dispersed in 1 g of gelatin.

Subsequently, a coating solution of an adhesive layer was prepared by dissolving 4 g of gelatin and 0.2 g of Nonion HS 210 in 100 ml of water. Thus prepared solution was coated over the light-shielding layer and dried to give an adhesive layer of 4 μm thick (dry basis).

The adhesive layer was then wetted with water in the amount of 30 g/m$^2$, and a cotton broadcloth (100% cotton, woven from cotton yarn of 100 count, manufactured by Toyobo Co., Ltd., Japan) was pressed onto the wetted adhesive layer and dried to give a porous spreading layer. Thus, a multilayer analytical element for quantitative analysis of glucose was prepared.

The analytical element was cut to obtain a square chip (1.5 cm×1.5 cm), which was in turn inserted into a plastic mount disclosed in Japanese Patent Provisional Publication No. 57(1982)-63452 to prepare an analytical slide for quantitative analysis of glucose.

Fresh blood was collected from a human body in the presence of a heparin and centrifuged at 3,000 rpm for two minutes to separate the plasma portion from the corpuscle portion. Both were then mixed in different ratios to prepare 10 blood samples (whole blood samples) having centrifugal hematocrit values ranging from 20% to 70%.

Each sample of thus prepared whole blood in the amount of 6 μl was spotted on the spreading layer of the analytical slide, which was then incubated at 37°C for 6 min, and the optical density of colored portion produced on the reagent layer was measured by reflection photometry through the transparent support. Subsequently, the analytical slide was measured on the red color density of a filmy residual whole blood remaining on the surface of the spreading layer by reflection photometry from the spreading layer side (wavelength for measurement: 500 nm).

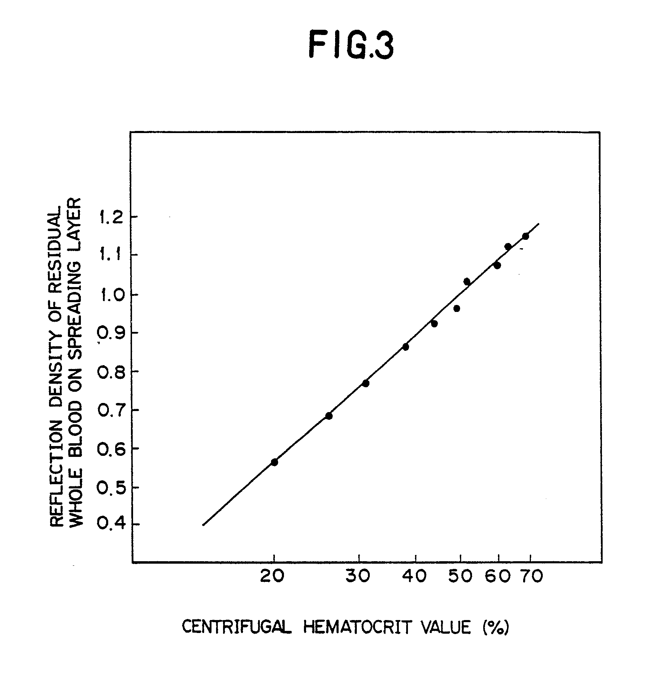

The logarithmic centrifugal hematocrit value and the reflection density of the residual whole blood on the spreading layer were placed on orthogonal coordinates, to give the graph of Fig. 3 in which a primary correlation was observed. According to the result, it is apparent that a hematocrit value (centrifugal hematocrit value) of a blood (whole blood) having unknown hematocrit value is obtainable from a reflection density of a residual filmy whole blood on the spreading layer (hereinafter, referred to as "residual surface OD").

Each of 82 heparin-containing fresh whole blood samples collected from a human body in the amount of 6 μl was spotted on the spreading layer of the abovementioned chemical analytical slide by means of a micropipet. The slide was then incubated at 37°C for 6 min, and the reflection density of colored portion on the reagent layer was measured by reflection photometry through the transparent support. The measured reflection density was converted into a glucose content value in blood using the predetermined calibration curve. The resulting glucose content value was chosen as the fundamental value.

The hexokinase method was applied to each of the above-mentioned fresh blood samples to determine the glucose content in a plasma for each sample. The correlation between the glucose content in the plasma determined by the hexokinase method (x) and the glucose content in the blood determined by the chemical analytical slide (y) is set forth in Table 1.

TABLE 1

| | |
|---|---|
| Recurrence formula | y=1.0087x–11.02 |
| Correlation coefficient | r=0.9889 |
| Variance from recurrence formula | Syx=16.6 |
| Number of tested samples | n=82 |

In addition, it was found that tested samples extremely deviating from the correlation were high hematocrit whole bloods having the centrifugal hematocrit values of approx. 50% or more. However, since

6

the residual surface OD of the whole blood on the spreading layer primarily correlates to the logarithmic centrifugal hematocrit value and, further, samples of high (or abnormally high) hematocrit value can be known from the residual surface OD value thereof, it is apparent that the extent of deviation from the correlation can be easily judged from the residual surface OD without separately measuring hematocrit value of the whole blood.

The chemical analytical slide having been measured to determine the reflection density of the colored portion of the reagent layer (thus determined value or a glucose content value derived from the determined value was chosen as the fundamental value) was immediately subjected to measurement on the residual surface OD of whole blood at 500 nm by means of a reflection optical densitometer. Thus determined reflection density was chosen as the supplementary value.

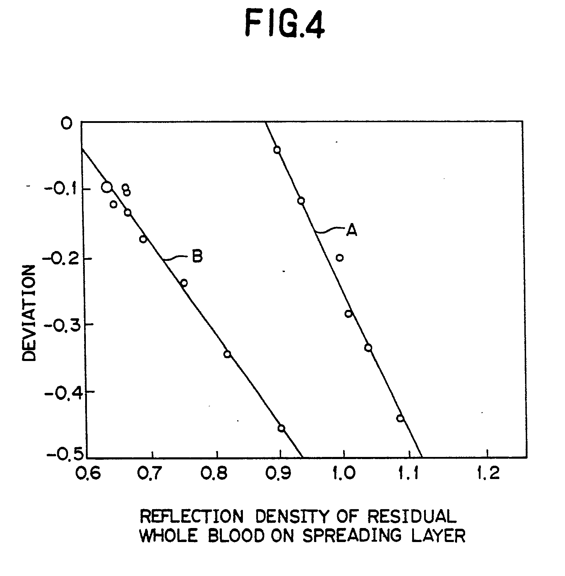

Along the horizontal (x) axis of orthogonal coordinates was plotted the residual surface OD value (supplementary value), while along the vertical (y) axis was plotted the deviation from the correlation, that is, (F−R)/R, in which F is the glucose content determined by means of the chemical analytical slide (fundamental value) and R is the glucose content determined by the hexokinase method. The result is given in Fig. 4 in which two satisfactorily straight correlation lines (line A for a sodium fluoride (NaF)-containing whole blood sample, and line B for a no-NaF-containing whole blood sample) are shown.

Based on the straight correlation lines shown in Fig. 4, the results of blood samples showing not less than (−) 1.0 of deviation from the correlation were subjected to calculation to obtain a corrected glucose content. The calculation was conducted according to the following equation:

Corrected glucose content=
(glucose content determined by means of
chemical analytical slide: fundamental value)
$\times(1/a\times$(residual surface OD)$+b+1)$

In the above equation, $(1/a\times$(residual surface OD)$+b+1)$ is a correction value derived from the residual surface OD of whole blood (supplementary value), and $a$ and $b$ correction constants set forth in the following table.

| Whole blood sample | $a$ | $b$ |
| --- | --- | --- |
| NaF contained | −2.503 | +2.263 |
| NaF not-contained | −1.330 | +0.748 |

The correlation between the glucose content determined by the hexokinase method (x) and the glucose content corrected as above (glucose content in plasma: y) is set forth in Table 2.

TABLE 2

| Recurrence formula | $y=1.0092x-6.16$ |
| --- | --- |
| Correlation coefficient | $r=0.9942$ |
| Variance from recurrence formula | $Syx=12.0$ |
| Number of tested samples | $n=82$ |

The results indicate that the correlation coefficient of the corrected glucose content is nearer to 1 than the uncorrected glucose content is, and that the variance from the recurrence formula decreases. Accordingly, it is apparent that in the analysis of a whole blood sample showing a hematocrit value deviating from the regular range the correlation to the glucose content value determined by the hexokinase method is remarkably improved.

Example 2

10 ml of venous blood was collected into a blood-collecting tube containing 25 mg of sodium fluoride (NaF: glycolysis inhibitor) and 150 U of heparin sodium. The glucose content in the blood was 118 mg/dl. The collected blood was lightly centrifuged at room temperature to separate the plasma portion from the corpuscle portion, and both were again mixed in certain ratios to prepare 6 samples (5 ml per each) having the same plasma glucose contents but different hematocrit values. Respective samples were named "whole blood samples Nos. 11—16". A certain portion of each sample was collected into a capillary tube and subjected to centrifuge at 3000 G for 5 min to determine the hematocrit value.

In the same manner, plural samples having different hematocrit values were prepared from a blood whose glucose content in blood was 254 mg/dl. Respective samples were name "whole blood samples Nos. 21—26".

7

A solution of a reagent layer having the composition set forth below for quantitative analysis of glucose contained in blood was coated over a transparent polyethylene terephthalate (PET) film (support, thickness: 185 μm) having a gelatin subbing layer, and the coated layer was dried to give a dry reagent layer of 15 μm thick.

Composition

| | |
|---|---|
| Peroxidase | 25,000 IU |
| 1,7-Dihydroxynaphthalene | 5 g |
| 4-Aminoantipyrine | 5 g |
| Gelatin | 200 g |
| Nonion HS 210 (trademark of Nippon Oil & Fats Co., Ltd., Japan, polyoxyethylene nonylphenyl ether surfactant) | 2 g |

On the reagent layer was provided a light-shielding layer of approx. 15 μm thick (dry basis) consisting of 8 g of powdery titanium dioxide carrying 0.2 g of Nonion HS 210 and 50,000 IU of glucose oxidase dispersed in 1 g of gelatin.

Subsequently, a coating solution of an adhesive layer was prepared by dissolving 4 g of gelatin and 0.2 g of Nonion HS 210 in 100 ml of water. Thus prepared solution was coated over the light-shielding layer and dried to give an adhesive layer of 4 μm thick (dry basis).

The adhesive layer was then wetted with water in the amount of 30 g/m², and a cotton broadcloth (100% cotton, woven from cotton yarn of 100 count, manufactured by Toyobo Co., Ltd., Japan) was pressed onto the wetted adhesive layer and dried to give a porous spreading layer. Thus, a multilayer analytical element for quantitative analysis of glucose was prepared.

The analytical element was cut to obtain a square chip (1.5 cm×1.5 cm), which was in turn inserted into a plastic mount disclosed in Japanese Patent Provisional Publication No. 57(1982)-63452 to prepare an analytical slide for quantitative analysis of glucose.

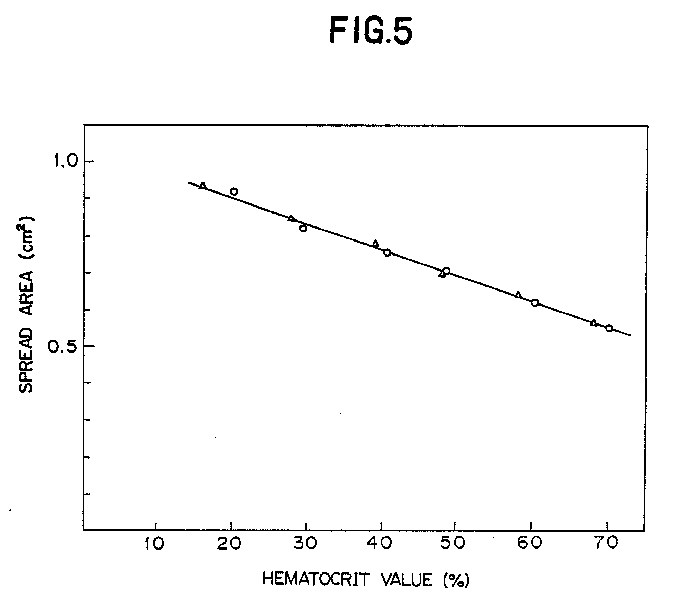

Each of the aforementioned whole blood samples (Nos. 11—16) in the amount of 6 μl was spotted on the spreading layer of the analytical slide, and the spread area on the spreading layer was measured after 2 min from the spotting side. The results are given in Fig. 5. It has been ascertained that a certain correlation resides between the spread area and the hematocrit value.

Each whole blood sample spotted was incubated at 37°C for 6 min. The optical density of colored portion on the reagent layer was measured by reflection photometry through the transparent support to give a fundamental value. As set forth in Table 3, the third column, the optical density of the colored portion varied with the hematocrit values.

In the fourth column, corrected values given by correcting the optical density of the colored portion on the reagent layer by applying thereto the correction value obtained from the correlation between the hematocrit value and the spread area shown in Fig. 5 are set forth.

It is understood that the blood sugar value corrected in the error brought about by influence of the hematocrit value can be obtained by correcting the optical density of the colored portion based on the correlation between the hematocrit value and the spread area of the whole blood sample on the spotted surface. It is further understood that the method of the present invention is very advantageously applied to the analysis of whole blood sample showing high hematocrit value deviating from the regular hematocrit range.

TABLE 3

| Whole blood sample No. | Hematocrit value (%) | Optical density of colored portion | |
|---|---|---|---|
| | | Uncorrected | Corrected |
| 11 | 16 | 0.462 | 0.463 |
| 12 | 28 | 0.467 | 0.468 |
| 13 | 39 | 0.665 | 0.666 |
| 14 | 48 | 0.452 | 0.461 |
| 15 | 58 | 0.423 | 0.458 |
| 16 | 68 | 0.388 | 0.455 |

8

## 0 112 166

Example 3

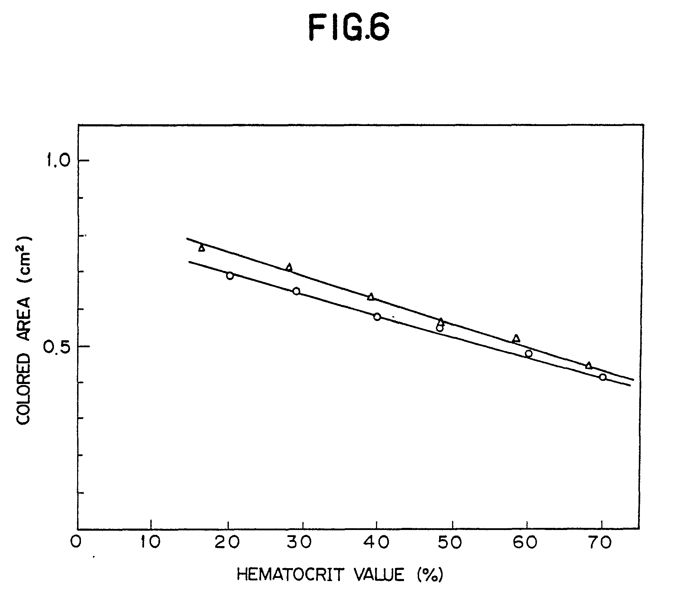

The procedures in Example 2 were repeated except that the colored area was measured through the transparent support at 7 min after the spotting. The correlation between the hematocrit value and the colored area is shown in Fig. 6. Based on the correlation, the measured optical density of the colored portion (fundamental value) was corrected in the same manner as in Example 2. The results are set forth in Table 4.

Accordingly, it has been ascertained that the error in the measure value brought about by the influence of the hematocrit value can be satisfactorily corrected by the correcting procedure utilizing the colored area measured from the transparent support side.

Example 4

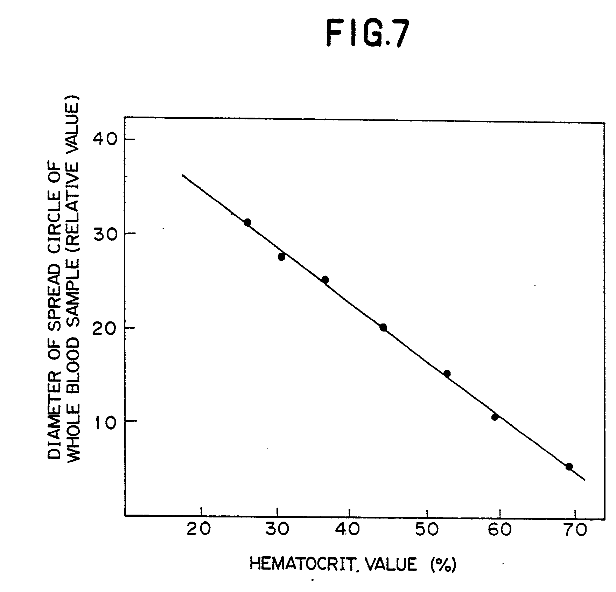

The procedures in Example 2 were repeated except that a diameter of a spread circle of the spotted whole blood sample was measured in place of the spread area of the spotted surface. A certain correlation was observed between the hematocrit value and the diameter of the spread circle as shown in Fig. 7.

Based on the correlation, the measured value (fundamental value) of Example 3 was corrected. The results are set forth in Table 4.

Accordingly, it has been ascertained that the error in the measured value brought about by the influence of the hematocrit value can be satisfactorily corrected by utilizing the diameter of the spread circle.

Example 5

The procedures in Example 4 were repeated except that a diameter of the circular colored area was measured through the transparent support in place of the measurement of the diameter of the spread circle on the spotted surface. There was observed the same correlation between the hematocrit value and the diameter of the circular colored area as that shown in Fig. 6.

Based on the correlation, the measured value (fundamental value) of Example 3 was corrected. The results are set forth in Table 4.

Accordingly, it has been ascertained that the error in the measured value brought about by the influence of the hematocrit value can be satisfactorily corrected by utilizing the diameter of the colored area measured from the transparent support side.

TABLE 4

| Whole blood sample No. | Hemato-crit value (%) | Optical density of colored portion | | | |
|---|---|---|---|---|---|
| | | | Corrected | | |
| | | Uncorrected | Example 3 | Example 4 | Example 5 |
| 21 | 20 | 0.767 | 0.765 | 0.771 | — |
| 22 | 29 | 0.770 | 0.761 | 0.759 | — |
| 23 | 40 | 0.759 | 0.759 | 0.748 | — |
| 24 | 48 | 0.738 | 0.757 | 0.742 | — |
| 25 | 60 | 0.709 | 0.751 | 0.735 | — |
| 26 | 70 | 0.697 | 0.747 | 0.727 | — |
| 31 | 19 | 0.864 | 0.866 | — | 0.863 |
| 32 | 28 | 0.869 | 0.870 | — | 0.865 |
| 33 | 40 | 0.853 | 0.861 | — | 0.856 |
| 34 | 48 | 0.800 | 0.858 | — | 0.849 |
| 35 | 58 | 0.773 | 0.855 | — | 0.832 |
| 36 | 70 | 0.733 | 0.851 | — | 0.827 |

Example 6

The reflection density of the red blood corpuscle residing in the spread circle on the spreading layer of the same chemical analytical slide for quantitative analysis of glucose as those employed in Examples 2—5 was measured on the spotted surface at 2 min after spotting of the sample by means of an analyzer equipped directly with a reflection optical densitometer (densitometer for prescale use, manufactured by Fuji Photo Film Co., Ltd., Japan). The optical density of colored portion of the reagent layer measured from the transparent support side in the same manner as in Example 2 (fundamental value) was corrected by utilizing the correlation between the hematocrit value and the reflection density previously determined in the manner described in Example 5.

Accordingly, it has been ascertained that the error in the measured value brought about by the influence of the hematocrit value can be satisfactorily corrected by utilizing the optical density of the red blood corpuscle color portion in the spread circle measured from the spreading layer side.

Example 7

On the PET film (support) were provided the reagent layer, light-shielding layer, and adhesive layer in this order in the same manner as in Example 2. Subsequently, a circular membrane filter (diameter: 9 mm, mean pore diameter: 5 μm, thickness: 165 μm) was laminated under adhesion on the adhesive layer in the manner as described in Japanese Utility Model Provisional Publication No. 57(1982)-42951 to prepare a multilayer analytical element for quantitative analysis of glucose provided with a porous patch having a definite surface area. Thus prepared multilayer analytical element was an analytical element showing a definite spreading propery in which a spread area was limited to correspond to the area of the bottom surface of the patch regardless of variation of the hematocrit value of whole blood samples.

The multilayer analytical element was cut to give a square chip (15 mm×15 mm) in which the patch was positioned in the center, and the chip was inserted into a plastic mount disclosed in Japanese Patent Provisional Publication No. 57(1982)-63452 in such a manner that the center of the patch was located in the center of the circular opening of the upper surface of the mount. Thus, an analytical slide for quantitative analsis of glucose was prepared.

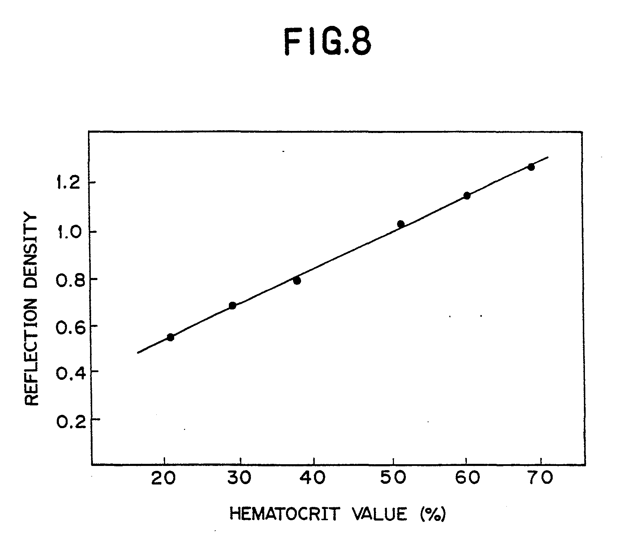

Each of whole blood samples prepared in the manner as in Examples 2—5 to show different hematocrit values was spotted on the patch of the above-prepared analytical slide in the amount of 8 μl. The slide was then subjected to incubation at 37°C for 6 min and measured on the optical density of colored portion of the reagent layer by reflection photometry from the transparent support side. Then, each slide was immediately measured on the optical density of the filmy layer consisting essentially of red blood corpuscle residing on the patch by reflection photometry from the patch side. A certain correlation was observed between the hematocrit value and the reflection density of the red blood corpuscle layer as shown in Fig. 8.

The optical density of colored portion of the reagent layer measured from the transparent support side was corrected utilizing the above-mentioned correlation to give a result satisfactorily corrected on the error brought about by influence of the hematocrit value. Accordingly, it has been ascertained that the.error of the measured value brought about by influence of the hematocrit value can be satisfactorily corrected utilizing the reflection density obtained by measurement of the filmy layer consisting essentially of red blood corpuscle residing on the patch.

Example 8

An analytical slide for quantitative analysis of glucose was prepared in the same manner as in Example 7 except that the patch having a definite surface area was replaced with a circular cotton broadcloth (diameter: 9 mm, thickness: approx. 200 μm).

On the patch of the slide was spotted a whole blood sample showing 42% of hematocrit value and 113 mg/dl of glucose content in an amount varying from 4 μl to 12 μl. The reflection density was measured on the spotted surface of the patch in the same manner as in Example 7. It was observed that the spotted amount and the reflection density was correlated linearly.

Accordingly, it has been ascertained that the error brought about into the optical density of colored portion of the reagent layer measured through the transparent support (fundamental value) by influence of the spotted amount can be satisfactorily corrected utilizing the above-mentioned correlation.

Example 9

A blood collected in the same manner as in Example 2 was centrifuged to prepare a plasma. Human albumin was added to the plasma to prepare a plasma sample containing protein in amount varying from 2 to 15 g/dl.

10 μl of the plasma sample was spotted on the spreading layer of the multilayer analytical element for quantitative analysis of glucose prepared in the manner as disclosed in Japanese Patent Provisional Publication No. 55(1980)-164356. The area of colored portion of the reagent layer was subsequently measured through the transparent support. It was noted that the correlation observed in Example 3 also resided between the total protein content and the colored area.

Accordingly, it has been ascertained that the error brought about into the optical density of colored portion of the reagent layer measured through the transparent support (fundamental value) by influence of the total protein content can be satisfactorily corrected by applying to the measured value a certain correction value obtained from the colored area of the reagent layer measured through the transparent support.

Example 10

A solution of a reagent layer having the composition set forth below was coated over a transparent PET film (support, thickness: 180 μm) having a gelatin subbing layer, and the coated layer was dried to give a dry reagent layer 20 μm thick.

| Composition | Part(s) by weight |
|---|---|
| Glucose oxidase (100 U/mg) | 2 parts |
| Peroxidase (150 U/mg) | 1 part |
| 1,7-Dihydroxynaphthalene | 5 parts |
| 4-Aminoantipyrine | 5 parts |
| Gelatin | 200 parts |
| Nonion HS 210 (trademark of Nippon Oil & Fats Co., Ltd., Japan, polyoxyethylene nonylphenyl ether surfactant) | 2 parts |

The above-prepared layer was then wetted with water, and a membrane filter (mean pore diameter 5 μm, Fuji Microfilter FM-500 manufactured by Fuji Photo Film Co., Ltd., Japan) was pressed onto the wetted layer and dried to give a multilayer analytical element for quantitative analysis of glucose.

The analytical element was then inserted into a plastic mount in the same manner as in Example 1 to prepare a chemical analytical slide.

On the spreading layer of the chemical analytical slide was spotted 10 μl of a serum sample varying in protein content prepared in the same manner as in Example 9 to examine a relationship between the protein content and the diameter of the spread circle.

It has been ascertained that correlation between the blood sugar value and the optical density of the colored portion can be prominently improved on the analysis of serum samples having different protein contents by correcting the reflection density of the reagent layer measured through the transparent support based on the above-mentioned relationship.

Example 11

An integral multilayer analytical element for quantitative analysis of blood urea nitrogen (BUN) was prepared in the manner described below.

On a colorless, transparent PET film (support, thickness: 180 μm) were provided in order the following layers to be coated in the indicated amounts per 1 $m^2$.

(1) Indicator layer

A solution of the following composition was employed.

| Dye precursor: 4-[bis-(2,4-dinitrophenyl)-methyl]-N-hexadecylpyridinium perchlorate | 1.00 g |
|---|---|
| Cellulose acetate | 8 g |
| Acetone | 65 ml |
| Methylcellosolve | 35 ml |
| Ethanesulfonic acid | 25 μl |

(2) Liquid-blocking layer

A membrane filter (Fuji Microfilter FM-500: trade name, thickness: 140 μm, void ratio: 75%, mean pore diameter: 5 μm) was dipped in a hexane solution containing silicone resin and then dried so that the membrane filter was made water-repellent with the silicone resin. Thus treated membrane filter was pressed to adhere to the indicator layer (1) not yet dried (under wet conditions) and then dried.

(3) Reagent layer

A solution of the composition given below was adjusted to pH 8 with a combination of disodium orthophosphate and sodium hydroxide, coated over the membrane filter, and dried.

11

| | |
|---|---|
| Gelatin | 10 g |
| Water | 100 ml |
| p-Nonylphenoxypolyglycidol (Glycidol Surfactant 10G) | 0.30 g |
| Urease | 0.8 g |
| Tetrasodium ethylenediaminetetraacetate | 0.4 g |

(4) Light-reflecting layer

A solution of the following composition was coated over the reagent layer and dried.

| | |
|---|---|
| $TiO_2$ fine powder | 4 g |
| Gelatin | 4 g |
| p-Nonylphenoxypolyglycidol | 0.15 g |
| Water | 40 g |

(5) Adhesive layer

A solution of the following composition was coated over the light-reflecting layer and dried. The adhesive layer was provided to assist provision of a spreading layer on the light-reflecting layer under adhesion.

| | |
|---|---|
| Gelatin | 2.5 g |
| Water | 50 ml |
| p-Nonylphenoxypolyglycidol | 0.15 g |

(6) Spreading layer

The surface of the dry adhesive layer was wetted with water to swell, and on this surface was laminated a cotton broadcloth (#100) under pressure to provide a spreading layer.

The multilayer analytical element prepared as above was converted into a chemical analytical slide for quantitative analysis of BUN in the same manner as in Example 2.

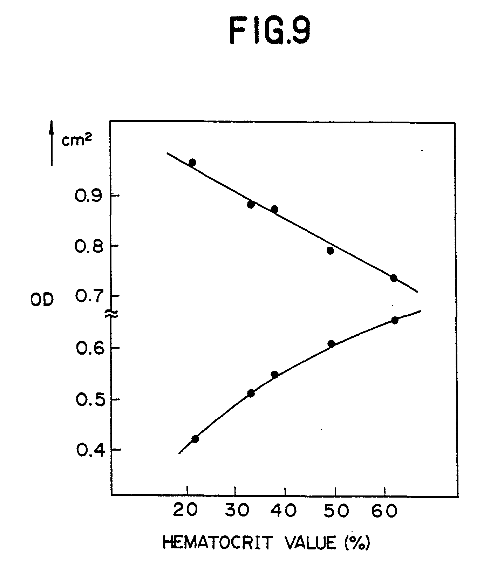

The same blood sample as in Example 2 was applied onto the above-mentioned chemical analytical slide to examine a correlation between the hematocrit value and the reflection density of the colored portion measured through reflection photometry from the transparent support side. The result is given in Fig. 9 on the lower side curve. Further, a correlation between the hematocrit value and the area of the colored portion measured through the transparent support was examined. The result is given in Fig. 9 on the upper side curve.

Accordingly, it has been ascertained that the error brought into a measured optical density of a whole blood sample (fundamental value) by influence of the hematocrit value can be reduced to ±5% or less by correcting the fundamental value based on the correction value determined from the correlation between the two curves.

Example 12

A chemical analytical slide for quantitative analysis of blood bilirubin was prepared in the manner described in Example 2 of Specification of Japanese Patent Provisional Publication No. 57(1982)-37262.

Blood samples having differnet hematocrit values were prepared in the same manner as in the aforementioned Example 2. The blood sample was then applied to the above-mentioned chemical analytical slide, and the reflection density of color produced by the reaction of bilirubin and received in the bilirubin-receiving layer was measured by reflection photometry through the transparent support. Independently, the reflection density of the surface of the spreading layer was measured to examine a correlation between the reflection density and the hematocrit value.

It has been ascertained that the error brought about into the measured value on the bilirubin content in the whole blood sample by influence of the hematocrit value can be corrected to a similar extent achieved in the quantitative analysis of glucose on the aforementioned chemical analytical element, utilizing a certain correction value determined from the correlation.

Example 13

A chemical analytical slide for quantitative analysis of blood sugar was prepared in the manner described in Example 1 of Specification of Japanese Patent Provisional Publication No. 55(1980)-164356.

Blood samples having different hematocrit values were prepared in the same manner as in the previously mentioned Example 2. An ultrasonic wave was applied to the blood sample having different hemoglobin contents. Using the hemolytic blood sample, the procedure described in the Example 2 was repeated to examine a correlation between the optical density of the formed color (fundamental value) and the hematocrit value.

Accordingly, it has been ascertained that the error brought about into the fundamental value on the analysis of a variety of hemolytic whole blood samples by influence of the hematocrit value can be corrected, utilizing a certain correction value determined from the correlation.

12

**Claims**

1. A method for quantitative analysis of an analyte contained in a blood (i.e. whole blood, plasma or serum) sample which comprises applying a certain amount of a blood sample onto an analytical element in the form of a sheet comprising a reagent-retaining layer provided on a transparent support and photometrically measuring a change of color density or a rate of the change of color density from the transparent support side to determine quantitatively the amount of the analyte in the blood sample, characterised in that the method comprises the steps of:

choosing, as a fundamental value, a value determined by measuring photometrically from the transparent support side the change of color density or the rate of the change of color density produced on the analytical element by the application of the blood sample;

choosing and determining, as a supplementary value, at least one value selected from a value obtained by photometrically measuring a color density or a shape of the colored portion on the surface of the same element onto which the blood sample has been applied, and a value obtained by photometrically measuring a shape of the colored portion from the transparent support side; and

correcting said fundamental value by applying thereto a correction value determined from a predetermined relationship between the supplementary value and a factor of the blood sample influencing the supplementary value and the fundamental value.

2. The method as claimed in Claim 1, in which determining the fundamental value is preformed prior to determining the supplementary value.

3. The method as claimed in Claim 1, in which determining the fundamental value and determining the supplementary value are performed substantially at the same time.

4. The method as claimed in Claim 1, in which determining the supplementary value is preformed prior to determining the fundamental value.

5. The method as claimed in Claim 1, in which the analytical element comprises a spreading layer provided on the reagent-retaining layer and the blood sample is applied onto said spreading layer.

6. The method as claimed in Claim 5, in which the analytical element comprises a light-shielding layer provided between the reagent-retaining layer and the spreading layer.

7. The method as claimed in Claim 1, in which the analytical element comprises a patch having a definite surface area and the blood sample is applied onto said patch.

8. The method as claimed in Claim 7, in which the analytical element comprises a light-shielding layer provided between the reagent-retaining layer and the patch.

**Patentansprüche**

1. Verfahren zur quantitativen Analyse einer zu analysierenden Substanz (Analyt), die in einer Blutprobe (unbehandeltes Blut, Blutplasma oder Blutserum) enthalten ist, bei welchem eine bestimmte Menge einer Blutprobe auf ein analytisches Element in der Form eines Blattes aufgebracht wird, das mit einer reagenshaltenden Schicht auf einem transparenten Träger versehen ist, und eine Änderung der Farbdichte oder eine Änderungsrate der Farbdichte von der transparenten Trägerseite aus photometrisch gemessen wird, um quantitativ die Menge des Analyts in der Blutprobe zu messen, dadurch gekennzeichnet, daß das Verfahren folgende Schritte enthält: Das Wählen eines Wertes, als Grundwert, der durch photometrisches Messen der Änderung der Farbdichte oder der Änderungsrate der Farbdichte, die durch das Aufbringen der Blutprobe auf das analytische Element erzeugt wird, von der transparenten Trägerseite aus durchgeführt wird; Wählen und Bestimmen wenigstens eines Wertes, als Zusatzwert, aus einem Wert, der durch photometrisches Messen einer Farbdichte oder einer Form des gefärbten Teils der Oberfläche desselben Elementes, auf das die Blutprobe aufgebracht wurde, erhalten wird, und aus einem Wert, der durch photometrisches Messen einer Form des gefärbten Teils von der transparenten Trägerseite aus erhalten wird; und Korrigieren des Grundwertes durch Beifügen eines Korrekturwertes, der aus einer vorbestimmten Beziehung zwischen dem Zusatzwert und einem Faktor der Blutprobe bestimmt wird, welcher den Zusatzwert und den Grundwert beeinflußt.

2. Verfahren nach Anspruch 1, bei dem das Bestimmen des Grundwertes vor dem Bestimmen des Zusatzwertes durchgeführt wird.

3. Verfahren nach Anspruch 1, bei dem das Bestimmen des Grundwertes und das Bestimmen des Zusatzwertes im wesentlichen zur gleichen Zeit durchgeführt werden.

4. Verfahren nach Anspruch 1, bei dem das Bestimmen des Zusatzwertes vor dem Bestimmen des Grundwertes durchgeführt wird.

5. Verfahren nach Anspruch 1, bei dem das analytische Element eine auf der reagenhaltenden Schicht vorgesehene Verteilerschicht enthält und die Blutprobe auf die Verteilerschicht aufgetragen wird.

6. Verfahren nach Anspruch 5, bei dem das analytische Element zwischen der reagenshaltenden Schicht und der Verteilerschicht eine lichtabschirmende Schicht enthält.

7. Verfahren nach Anspruch 1, bei dem das analytische Element einen ein bestimmtes Oberflächengebiet einnehmenden Bereich enthält und die Blutprobe auf diesen Bereich aufgetragen wird.

8. Verfahren nach Anspruch 7, bei dem das analytische Element eine zwischen der reagenshaltenden Schicht und dem Bereich vorgesehene lichtabschirmende Schicht enthält.

# 0 112 166

**Revendications**

1. Procédé d'analyse quantitative d'une substance à analyser contenue dans un échantillon de sang (c'est-à-dire de sang complet, de plasma ou de sérum), dans lequel on applique une certaine quantité d'un échantillon de sang sur un élément analytique sous la forme d'une feuille comprenant une couche de rétention d'un réactif, prévue sur un support transparent et on mesure par photométrie une variation de densité de couleur ou une vitesse de variation de densité de couleur à partir du côté du support transparent, afin de déterminer quantitativement la quantité de la substance à analyser dans l'échantillon de sang, caractérisé en ce qu'il comprend les étapes consistant à choisir, en tant que valeur fondamentale, une valeur déterminée en mesurant par photométrie, à partir du côté du support transparent, la variation de la densité de couleur ou la vitesse de la variation de densité de couleur produite sur l'élément analytique par l'application de l'échantillon de sang, à choisir et déterminer, en tant que valeur supplémentaire, au moins une valeur choisie parmi une valeur obtenue en mesurant par photométrie une densité de couleur ou une forme d'une portion colorée sur la surface du même élément sur lequel l'échantillon de sang a été appliqué, et une valeur obtenue en mesurant par photométrie une forme de la portion colorée à partir du côté support transparent, et à corriger ladite valeur fondamentale en appliquant à celle-ci une valeur de correction déterminée à partir d'une relation prédéterminée entre la valeur supplémentaire et un facteur de l'échantillon de sang influençant la valeur supplémentaire et la valeur fondamentale.

2. Procédé suivant la revendication 1 caractérisé en ce qu'on détermine la valeur fondamentale avant de déterminer la valeur supplémentaire.

3. Procédé suivant la revendication 1 caractérisé en ce qu'on détermine la valeur fondamentale et on détermine la valeur supplémentaire pratiquement en même temps.

4. Procédé suivant la revendication 1 caractérisé en ce qu'on détermine la valeur supplémentaire avant de déterminer la valeur fondamentale.

5. Procédé suivant la revendication 1 caractérisé en ce que l'élément analytique comprend une couche étalée prévue sur la couche de rétention du réactif et l'échantillon de sang est appliqué sur cette couche étalée.

6. Procédé suivant la revendication 5 caractérisé en ce que l'élément analytique comprned une couche formant écran à l'égard de la lumière et qui est prévue entre la couche de rétention du réactif et la couche étalée.

7. Procédé suivant la revendication 1 caractérisé en ce que l'élément analytique comprend une pastille ayant une aire superficielle définie et l'échantillon de sang est appliqué sur cette pastille.

8. Procédé suivant la revendication 7 caractérisé en ce que l'élément analytique comprend une couche formant écran à l'égard de la lumière et qui est prévue entre la couche de rétention du réactif et la pastille.

14

# FIG.1

# FIG.2

# FIG.3

CENTRIFUGAL HEMATOCRIT VALUE (%)

REFLECTION DENSITY OF RESIDUAL WHOLE BLOOD ON SPREADING LAYER

# FIG.4

REFLECTION DENSITY OF RESIDUAL
WHOLE BLOOD ON SPREADING LAYER

# FIG.5

# FIG.6

# FIG.7

# FIG.8

# FIG.9